Europäisches Patentamt

**(19)** **European Patent Office**

**Office européen des brevets**

(11) Publication number: **0 099 757**

**A2**

(12) # EUROPEAN PATENT APPLICATION

(21) Application number: **83304190.8**

(22) Date of filing: **19.07.83**

(51) Int. Cl.³: **A 61 B 5/00**
**G 01 N 21/31**

(30) Priority: **19.07.82 US 399866**

(43) Date of publication of application:
**01.02.84 Bulletin 84/5**

(84) Designated Contracting States:
**CH DE FR GB IT LI NL SE**

(71) Applicant: **Stoller, Milton**
**68 Hilldale Road**
**West Hartford Connecticut(US)**

(72) Inventor: **Stoller, Milton**
**68 Hilldale Road**
**West Hartford Connecticut(US)**

(74) Representative: **SERJEANTS**
**25, The Crescent**
**Leicester, LE1 6RX(GB)**

(54) **Diaphanoscopy apparatus.**

(57) In a diaphanoscopy apparatus a light source (10) is provided for illuminating the object with light having at least two different wavelengths, detector menas (15) are provided for sensing transilluminated light in relation to each of the different wavelengths; recording means (38,40) are provided for separately recording the output of the detector means (15) relating to the different wavelengths; and display means (66) are provided for producing a display which represents the absorption characteristics of the object at the different wavelengths.

0099757

-1-

<u>TITLE</u>:

Diaphanoscopy Apparatus

<u>DESCRIPTION</u>:

<u>Field of the Invention</u>

The invention relates to diaphanoscopy apparatus for use for example in non-destructive testing or

medical diagnostics.

<u>Background Art</u>

Transillumination, i.e., the passing of light through tissue, is also known as diaphanography or diaphanoscopy, and is utilised for diagnosing abnormalities.  Earlier apparatus and techniques are described in the U.S.

Patent Specification 3127115; 3371202; 3527932;

3674008; 3711700; 3732416; 3769963; 4077399; 4212306;

4286602; USSR Patent Specifications 279879 and 591178;

in "Diaphanalogie mammaire" by C. Gross <u>J</u>. <u>Radiol</u>.

<u>Electrol</u>. 1972 Vol 53 No. 4 pp 297-306 and "Etude dianoscopique des atterations dystrophiques du sein" by C. Maggio Senologia June 1978 pp69-71.

It is amongst the aims of the invention to provide improved diaphanoscopy apparatus.

## General Description of Invention

The invention provides firstly a diaphanoscopy apparatus which is characterised in that a light source is provided for illuminating the object with light having at least two different wave lengths, detector means are provided for sensing transilluminated light in relation to each of the different wave lengths, recording means are provided for separately recording the output of the detector means relating to the different wavelengths; and display means are provided for producing a display which represents the absorption characteristics of the object at the different wavelengths. The object examined may be human body tissue. Information commensurate with the light which passes through the tissue at each of a plurality of preselected illumination wavelengths can be stored. The stored information can be subsequently employed to produce an image which represents absorption characteristics of the tissue which has been illuminated.

Preferably an encoding means is provided for providing a characteristic value which is a function of both outputs of the detector means relating to the different wavelengths and the detector means is adapted for scanning so as to produce an image from a series of characteristic values for different parts of the object. The characteristic value may be a ratio of the different detector outputs and may be highly revealing of tissue abnormalities.

The apparatus includes a light source having at least two different wavelengths i.e. different frequencies. The term "wavelengths" is used in a general indicative fashion. The source can produce light lying within different pre-selected bands of frequency. The transilluminated light is split so as to separate the bands and measure absorption at different wavelengths. However the split light may share some part of the spectrum or may overlap but the spectral characteristics should be sufficiently different to give meaningful results. In accordance with one embodiment, the light source provides radiation which includes light having wavelengths lying in the red and near infrared regions. The tissue under examination is illuminated and the light which passes through the tissue is divided, separately filtered and sensed by a video system comprising a camera associated with each filter. The outputs of the cameras are a series of signals corresponding to images of the tissue as illuminated by the light at the different selected wavelengths. These video signals are separately stored and subsequently employed to form a composite image.

Also in accordance with the preferred embodiment, the information commensurate with the stored images is employed to produce a display wherein each point of the display contains information characteristic of the ratio of the light within each frequency band which has passed through the tissue at a corresponding point. Thus, by way of example, a colour may be assigned to each ratio and a multi-colour image formed.

The invention secondly provides a non-destructive testing method using diaphanoscopy in which an object is transilluminated with light characterised in that the transilluminating light has at least two different wavelengths, the transilluminated light is separated into beams each having different spectral compositions, the intensity of the different beams is sensed and separately recorded for a plurality of object locations, and a characteristic value is derived from a combination of the respective intensity values and displayed to provide an image representing the absorption of light on transillumination of the object at the different wavelengths.

Drawings

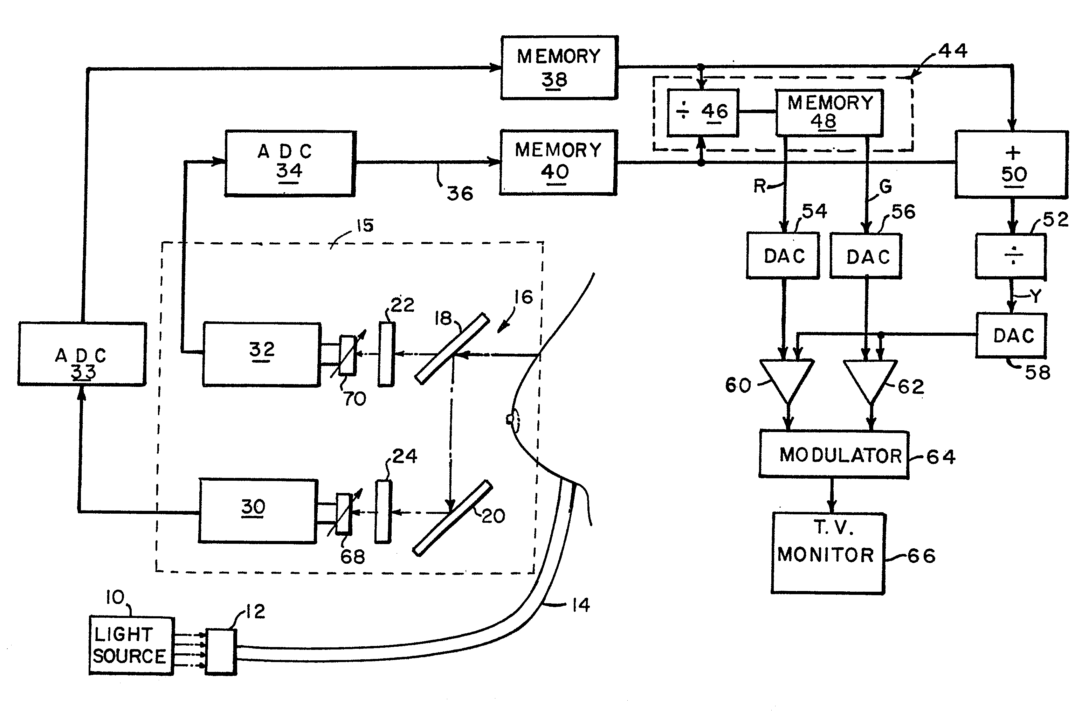

The Figure shows a functional block diagram of apparatus in accordance with the invention.

Description of the Preferred Embodiment

With reference now to the drawing, "white" light, i.e., light including wavelengths in the red and near infrared spectra, is employed for transilluminating human body tissue. Light at the different wavelengths chosen will be absorbed by the tissue as a function of wavelength and tissue type. The "white" light is generated by a lamp 10. The light produced by the lamp 10 is collected by a condensing lens system 12 which focuses the light at the end of a fibre optic bundle 14.

In examining a breast, as depicted in the drawing, the free end of the fibre optic bundle 14 is placed in contact with the skin to produce a source of transilluminating light. During transillumination a first element of a beam splitter, indicated generally at 16, is positioned so that light passing through the breast or other object being examined will fall on its face. The beam splitter 16 includes mirrors 18 and 20. One half of the light incident on the face of the mirror 18 which is directed toward the light source will pass through the mirror while the other half of the incident light will be reflected to the mirror 20. The light passing through mirror 18 is, in turn, passed through a filter 22. The light reflected from mirror 20 is directed through a filter 24. Filter 22, in the embodiment being described, will pass only wavelengths in the 650 - 750 nonometer, i.e. red, region. Filter 24 will pass light only in the 750 - 850 nonometer, i.e. near infrared, region.

Filters 22 and 24 are respectively positioned directly in front of the lens assemblies of respective video cameras32 and 30. The cameras 32 and 30 will include a silicon face plate tube that is responsive in the region from 650 nanometers to 900 nanometers. The camera tubes may, for example, comprise silicon diode array type devices available from RCA under the Trade Mark "ULTRICON". The light received by the cameras will have the effect of discharging, in varying degrees, the surface of the video camera tubes. Scanning of the tube surfaces by an electron beam produces, in the conventional manner, a video output signal. The analog output signals from cameras 32 and 30 are respectively delivered as the inputs to analog-to-digital convertors 34 and 33. The digitally coded signals from convertors 34 and 33 are supplied to respective frame memories 40 and 38. The memories 40 and 38 may, for example, comprise dynamic memory elements having 8 bits of memory for each picture location, i.e. each pixel. The operation of cameras 32 and 30 as well as the read out of memories 40 and 38 will be synchronized.

In operation of the system "red" light which is not absorbed by the object under examination will be passed by filter 22 to camera 32 and a signal commensurate with the intensity of the red light will be stored in the memory 40. Thus, the memory 40 will contain information commensurate with the degree of absorption of red light by, for example, the breast tissue at each point within the viewing field. Similarly, the light in the near infrared region which is passed through the test object will be delivered to the camera 30 and a signal commensurate therewith stored in the memory 38. The memory 38 will thus contain information commensurate with the degree of absorption of light in the near infrared region of the test object.

The information in memories 38 and 40 is simultaneously read by an encoder which is indicated generally at 44. In actual practice, encoder 44 will comprise a RAM which functions as a look-up table. To facilitate understanding of the disclosed embodiment of the invention, the encoder 44 has been functionally depicted as a divider 46 and a memory 48. The memory 48 will

have, for example, $2^8$ x $2^8$ addresses and numbers corresponding to the intensity of two colours, typically red and green, commensurate with ratios of the numbers which may be stored at each pixel in memories 38 and 40 will be stored at the memory locations in memory 48. The data stored in the memories 38 and 40 will be read by memory 48 at twice the rate of loading of the memories 38 and 40. The numbers stored at the corresponding memory locations in the memories 38 and 40 are employed to address memory 48. The memory 48 will produce a pair of colour related, digitally coded output signals for each pixel. This is functionally equivalent to dividing the numbers stored at the memory locations in memories 38 and 40 in the divider 46 and employing the thus produced ratio to address the memory 48. The numbers which are read out of the memory 48 comprise digitally coded chrominance signals which, in the example being described, will correspond to a red "R" intensity and a green "G" intensity.

The numbers read from memories 38 and 40 are also applied to an adder 50 where they are summed. The output of the adder 50 is delivered to a divide by two circuit 52. The output of the divider 52 is a digital encoded average luminance or "Y" signal.

The "R" and "G" chrominance signals from the memory 48 are converted to analog form by means of digital-to-analog converters 54 and 56 while the average luminance signal is convered to analog form by digital-to-analog converter 58. The outputs of the converters 54 and 56 are respectively applied as first inputs to differential amplifiers 60 and 62. The second input to the amplifiers 60 and 62 is the luminance  signal from the converter 58. The combined luminance and chrominance signals appearing at the outputs of the amplifiers 60 and 62 are applied to a standard TV modulator 64. The modulator 64 provides a composite colour video signal which is delivered to a TV monitor 66. This composite signal will, in the customary fashion, provide horizontal sync, colour burst and colour modulation information for each frame.

It is to be noted that, in the interest of reducing the size of the look-up memory, the encoder 44 may look at the most significant six bits of the signals stored in memories 38 and 40 while the adder 50 will look at all eight bits of the stored data.

It is also to be noted that the invention may be employed as an analytical tool wherein the ratio of the absorption by the object under examination of light at the selected transillumination frequencies at any point of interest may be read out and displayed on monitor 66. The amount of absorption of different colour light varies with the nature of the tissue being transilluminated even in the case of normal tissue. Accordingly, it has been found desirable to initially perform a "normalizing" step when practising the invention. Since the absorption of light in the red and near infrared regions will vary as a function of whether the tissue under examination is glandular or fatty, an initial adjustment will typically be made so that the image of normal tissue will be in a pre-selected colour or colours. Thus, in a typical case, normal tissue will be displayed as a white and black image while abnormalities will be represented by colour. In accordance with the disclosed embodiment of the invention the above-discussed step of "normalizing" is achieved through use of the "f-stop" controls, indicated schematically at 68 and 70, which form standard portions of the lens assemblies of vidicons 30 and 32. As noted above, the "normalization" procedure will typically be performed so as to cause all normal tissue to appear in white on a black background. Thus, at the onset of each examination, the apparatus will be adjusted so that transillumination of the patient's normal tissue will result in cameras 30 and 32 receiving light of equal intensity during the illumination of the tissue.

CLAIMS:

1.    A diaphanoscopy apparatus having a means for transilluminating an object with light characterised in that a light source (10) is provided for illuminating the object with light having at least two different wavelengths, detector means (15) are provided for sensing transilluminated light in relation to each of the different wavelengths, recording means (38,40) are provided for separately recording the output of the detector means (15) relating to the different wavelengths; and display means (66) are provided for producing a display which represents the absorption characteristics of the object at the different wavelengths.

2.    A diaphanoscopy apparatus according to claim 1 further characterised in that an encoding means (44) is provided for providing a characteristic value which is a function of both outputs of the detector means (15) relating to the different wavelengths and the detector means (15) is adapted for scanning so as to produce an image from a series of characteristic values for different parts of the object.

3.    A diaphanoscopy apparatus according to claim 2 further characterised in that the encoding means (44) includes a means (46) for producing a ratio of the respective detector outputs as a characteristic signal and the detector means (15) includes a pair of television cameras (30,32) responsive to the different wavelengths.

4. A diaphanoscopy apparatus according to any of claims 2 or 3 further characterised in that means (18) split transilluminated light having at least two different wavelengths into at least two components with at least two spectral characteristics for separate detection and recordal.

5. A diaphanoscopy apparatus according to claims 2, 3 or 4 in which the detector means (15) are provided with a means (18,20) for dividing transilluminated light into at least two beams and a filter means (22,24) for filtering the respective beams to produce beams of different spectral ranges including the different wavelengths, and means (68,70) for regulating the intensity of at least one of the beams or the output produced thereby for the calibration.

6. A diaphanoscopy apparatus according to claim 5 further characterised in that the calibrating means (68,70) are shutter means for controlling the amount of light transmitted in at least one of the beams to provide a colour display, and in that means (44,50,52) are provided to process digitised intensity signals for the same object location to provide at least two chrominance signals and a common luminance signal for controlling the modulator (64)..

7. A diaphanoscopy apparatus according to any of claims 2 to 6 further characterised in that digitising means (33,34) are provided for converting the respective outputs of the detector means (15) and the recording means (38,40) are digital memory devices for storing data related to the intensity of the transilluminated light of different wavelengths for different scanned locations, and the display means (66) is a television monitor receiving inputs from a modulator (64).

8. A diaphanoscopy apparatus according to claim 7 further characterised in that the digital memory devices (38,40) have simultaneously addressable memory locations for the data pertaining to the transilluminated light of different wavelengths for the same scanned location and said data are subjected to division in a divider (46) for producing a pair of chrominance values and to an adder (50) and an averaging means (52) for producing a luminance value and in that the modulator (64) is arranged to receive said values converted to analogue form through amplifiers (60, 62).

9.   A diaphanoscopy apparatus according to claim 7 further characterised in that means are provided for assigning intensity levels for two different colours depending on the digital memory device contents for controlling the modulator (64).

10.   A non-destructive testing method using diaphanoscopy in which an object is transilluminated with light characterised in that the transilluminating light has at elast two different wavelengths, the transmilluminated light is separated into beams each having different spectral compositions, the intensity of the different beams is sensed and separately recorded for a plurality of object locations, and a characteristic value is derived from a combination of the respective intensity values and displayed to provide an image representing the absorption of light on transillumination of the object at the different wavelengths.